(19) Europäisches Patentamt
European Patent Office
Office européen des brevets

(11) Veröffentlichungsnummer : **0 118 794**
**B1**

(12) **EUROPÄISCHE PATENTSCHRIFT**

(45) Veröffentlichungstag der Patentschrift : **10.02.88**

(21) Anmeldenummer : **84101540.7**

(22) Anmeldetag : **15.02.84**

(51) Int. Cl.[4] : **C 07 D311/58**, C 07 D311/60, C 07 D311/64, C 07 D311/70, C 07 D493/04, A 01 N 43/16, A 01 N 43/24

# (54) Neue Chromen-Derivate.

(30) Priorität : **15.02.83 HU 50483**

(43) Veröffentlichungstag der Anmeldung : **19.09.84 Patentblatt 84/38**

(45) Bekanntmachung des Hinweises auf die Patenterteilung : **10.02.88 Patentblatt 88/06**

(84) Benannte Vertragsstaaten : **AT BE CH DE FR GB IT LI NL SE**

(56) Entgegenhaltungen :
**DE-A- 2 639 671**
**DE-A- 2 833 067**
**US-A- 4 162 326**
**NATURE, Band 281, 18. Oktober 1979, Seiten 570-572, MacMillan Journals Ltd., London, GB, G.T. BROOKS et al.: "The action of precocenes in milkweed bugs (Oncopeitus fasciatus) and locusts (Locusta migratoria)"**
**NATURE, Band 284, 27. März 1980, Seiten 320-323, MacMillan Journals Ltd., London, GB, G.E. PRATT et al.: "Lethal metabolism of precocene-I to a reactive epoxide by locust corpora allata"**
**Organisch-chemische Nomenklatur, Frobenius, Wissenschaftliche Verlagsgesellschaft mbH, Stuttgart 1983**

(73) Patentinhaber : **ALKALOIDA VEGYéSZETI GYáR**
**Postfach 1**
**H-4440 Tiszavasvári (HU)**

(72) Erfinder : **Timar, Tibor, Dr. Dipl. Ing. Chem.**
**Elmunkás u. 27**
**H-4440 Tiszavasvári (HU)**
Erfinder : **Zsupán, Kálmán, Dr. Dipl. Ing. Chem.**
**Elmunkás u. 23/a**
**H-4440 Tiszavasvári (HU)**
Erfinder : **Répási, János, Dr.**
**Elmunkás u. 1**
**H-4440 Tiszavasvári (HU)**
Erfinder : **Borsos geb. Safranek, Irén**
**Elmunkás u. 21**
**H-4440 Tiszavasvári (HU)**
Erfinder : **Kiss, István, Dr.**
**Zöldfa u. 4**
**H-6723 Szeged (HU)**
Erfinder : **Fodor, András, Dr.**
**Retek u. 2**
**H-6723 Szeged (HU)**
Erfinder : **Maròy, Péter, Dr.**
**Ürhajòs u. 5/a**
**H-6723 Szeged (HU)**

(74) Vertreter : **Patentanwälte Beetz sen. - Beetz jun. Timpe - Siegfried - Schmitt-Fumian**
**Steinsdorfstrasse 10**
**D-8000 München 22 (DE)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**EP 0 118 794 B1**

**Beschreibung** (für die Vertragsstaaten : BE, CH, DE, FR, GB, IT, LI, NL, SE)

Die Erfindung betrifft neue Chromenderivate der Formel I

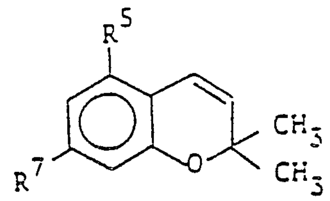

(I)

worin bedeuten :

$R^5$ Methyl oder Methoxy und

$R^7$ $C_{2-4}$-Alkoxy, $C_{3-5}$-Cycloalkoxy oder Propargyloxy,

ein Verfahren zu ihrer Herstellung sowie Schädlingsbekämpfungsmittel mit diesen Verbindungen als Wirkstoffen.

Die erfindungsgemäßen Verbindungen sind Analoga der Naturstoffe Precocen-1 (P1) und Precocen-2 (P2).

Die biologischen Wirkungen der Verbindungen P1 und P2 sind in der Literatur beschrieben (Bowers, W.S., et al., Sciences 193 (1976) 542 ; Brooks, G.T., et al., Nature 281 (1979) 570-572). Aufgrund ihrer biologischen Wirkung auf Insekten können diese Verbindungen als neue, umweltschonende Schädlingsbekämpfungsmittel verwendet werden. Die biologische Wirksamkeit, der Wirkungsmechanismus und der Metabolismus der Precocene sind in zahlreichen Publikationen beschrieben, wonach ihre Wirkung auf der Schädigung der Corpora allata der Insekten, welche die Juvenilhormone bilden, beruht. Ferner wurde bestimmt, wie die biologische Wirkung vom 2H-Chromenring abhängig ist. Bei den Untersuchungen wurde festgestellt, daß die Art und Stärke der Wirksamkeit der Precocene stark von der Art der Schädlinge, von den Testmethoden und, hinsichtlich der chemischen Struktur, von der Art und Anzahl sowie Stellung der Substituenten des aromatischen Rings, der Stärke der Doppelbindung im Pyranring sowie von den elektronischen und sterischen Parametern des ganzen Moleküls abhängig ist (Bowers, W.S., Martinez, P.R., Science 197 (1977) 1369 ; Schooneveld, H., Experientia 35 (1979) 363 ; Bowers, W.S., Pontif. Acad. Sci. Scr. Varia 41 (1976) 129 ; Brooks, C.T., et al., Nature 281 (1979) 570 ; Ohta, T., Kagaku to Seibutsu 17 (2) (1979) 92 ; Ohta, T., Konchu no Seiri to Kagaku (1979) 63 ; Matolcsy, G., et al., Z. Naturforsch. 35b (1980) 1449 ; Brooks, G.T., et al., Proc. Br. Crop. Prot. Conf. Pests. Dis. 1 (1979) 273 ; Pratt, G.E., et al., Nature 284 (1980) 320 ; Soderlund, D.M., et al., J. Agr. Food Chem: 28 (4) (1980) 724 ; Schooley, D.A., et al., Pestic. Biochem. Physiol. 13 (2) (1980) 95 ; Ottridge, A.P., et al., Pestic. Sci. 12 (3) (1981) 245 ; Sci. Papers of the Inst. of Org. and Phys. Chem. of Wroclaw Techn. Univ. 22 (7) (1981) 309-424 ; Pratt, G.E., Brooks, G.T. ed., Juvenile Hormone Biochemistry, Elsevier/North-Holland Biomed Press (1981) 311-437.

Im Rahmen dieser Untersuchungen wurden Derivate, wie 6-Methoxy-7-ethoxy-2,2-dimethyl-2H-chromen (P3) und 6-Methoxy-7-isopropoxy-2,2-dimethyl-2H-chromen, synthetisiert, die deutliche größere Aktivität zeigen als die in der Natur vorkommenden Precocene P1 und P2. Die Anzahl der bisher synthetisierten Precocenanaloga ist jedoch gering.

Es wurden auch mehrere, praktisch voneinander unabhängige Synthesen zur Herstellung von 2H-Chromenen angegeben (Chromenes, Chromanones and Chromones, G.P. Ellis ed., John Wiley and Sons, London (1977) 43-66 ; DE-PS 26 39 671, US-PS 4 162 326, JP-PSen 73121/79, 15411/80, 40637/80 und 43039/80, ES-PSen 496 301 und 496 302).

Eine bessere Kenntnis des Zusammenhangs zwischen Struktur und Wirkung würde zu einer Reihe von Verbindungen mit hoher Wirkung führen. Bis jetzt wurde jedoch die Anti-Juvenilhormon-Aktivität bei höchstens 30 bis 50 Verbindungen und nur bei 1 bis 2 Insektenarten bestimmt. Über ihre insecticide Wirkung, die sterilisierende, oocide sowie diapauseninduzierende Wirkung liegen fast keine Informationen vor.

Der Erfindung liegt entsprechend die Aufgabe zugrunde, neue Chromenderivate, die Precocenanaloga darstellen, mit hoher insecticider sowie nematocider Wirkung und ein Verfahren zu ihrer Herstellung sowie entsprechende Schädlingsbekämpfungsmittel anzugeben.

Die Aufgabe wird anspruchsgemäß gelöst. Vorteilhafte erfindungsgemäße Precocenanaloga sind Gegenstände der abhängigen Ansprüche.

Erfindungsgemäße Chromenderivate mit besonders günstigen Eigenschaften besitzen die Formel II

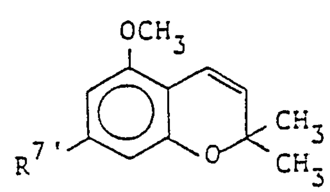

(II)

in der $R^{7'}$ n-Propoxy, i-Propoxy, s-Butoxy oder i-Butoxy bedeutet, sowie die Formel III

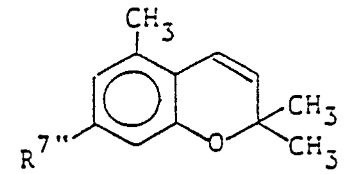

(III)

in der $R^{7''}$ Ethoxy, i-Propoxy, s-Butoxy oder n-Cyclopentyloxy bedeutet.

Das erfindungsgemäße Verfahren zur Herstellung der Chromenderivate der Formel I ist gekennzeichnet durch Cyclisierung von Arylpropargylethern der Formel IV

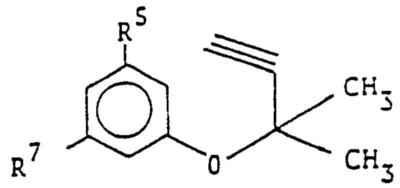

(IV)

in der

$R^5$ Methyl oder Methoxy und

$R^7$ $C_{2-4}$-Alkoxy, $C_{3-5}$-Cycloalkoxy oder Propargyloxy

bedeuten.

Die Arylpropargylether der Formel IV, aus denen die erfindungsgemäßen Chromenderivate der Formeln I, II und III durch Cyclisierung zugänglich sind, werden durch Umsetzung von Phenolen der Formel V

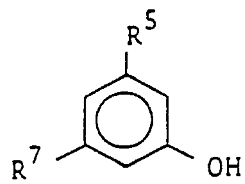

(V)

mit $R^5$ und $R^7$ wie oben mit Acetylenderivaten der Formel VI

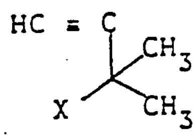

(VI)

mit X = Halogen hergestellt.

Diese Reaktion ist an sich bekannt (J. Hlubucek, E. Ritchie, W.C. Taylor, Aust. J. Chem. 11 (1971) 2347 ; R. Chenevert et al., Experientia 36 (1980) 379).

Das erfindungsgemäße Verfahren kann vorzugsweise wie folgt durchgeführt werden : Die Phenole der Formel V werden mit den Acetylenderivaten der Formel VI in Gegenwart von Basen, in Gegenwart von Alkaliiodiden als Katalysatoren sowie in Gegenwart von bipolaren aprotischen Lösungsmitteln zu den Arylpropargylethern der Formel VI umgesetzt. Ihre Cyclisierung erfolgt mit N,N-Dialkylarylaminen.

Die erfindungsgemäßen Schädlingsbekämpfungsmittel enthalten als Wirkstoffe Verbindungen der Formel I sowie übliche feste Trägerstoffe und/oder flüssige Verdünnungsmittel, wie Füllstoffe, Netzmittel, Dispergiermittel und/oder Emulgiermittel und/oder grenzflächenaktive Stoffe.

Die Erfindung wird im folgenden anhand von Beispielen näher erläutert.

Die Reinheit der erhaltenen Verbindungen wurde durch Dünnschichtchromatographie und Gaschromatographie, ihre Struktur durch IR-, NMR- und Massenspektrometrie überprüft.

Beispiel für die Angabe von NMR Spektren :

1,45 (3H ; t ; J = 5 Hz) : darin bedeuten :

1,45 — die chemische Verschiebung

3H — die Anzahl der Protonen

t — die Multiplizität
J — die Kopplungskonstante.

Bezeichnung der Multipletts :
s — Singulett
d — Dublett
t — Triplett
q — Quartett
m — größeres Multiplett
b — breite Spitze

## Beispiel 1

2,2-Dimethyl-5-methyl-7-propargyloxy-2H-chromen

20 mmol 2,2-Dimethyl-5-methyl-7-propargyloxy-chromanon werden in 100 ml eines Gemischs von Tetrahydrofuran/Wasser (1 : 1) gelöst und mit 5,3 g (30 mmol) Palladiumchlorid und 8,31 g (220 mmol) Natriumtetrahydroborat bei 0 °C 3 Stunden gerührt. Danach wird abgesaugt, das Filtrat dreimal mit je 100 ml Ether extrahiert und das Lösungsmittel entfernt. Der Rückstand wird in 300 ml Dichlormethan gelöst und mit 15 ml Pyridin und 2 g Phosphoroxychlorid 1 Stunde auf 35 °C erwärmt. Der Ansatz wird dann auf 100 ml Wasser gegossen und mit 5 % Salzsäure und mit Wasser gewaschen. Die organische Phase wird getrocknet ; nach Abtrennung des Lösungsmittels wird der Rückstand aus Methanol umkristallisiert. Ausbeute : 3,8 g (60 %), Fp. : 80-85 °C.

## Beispiel 2

7-Cyclopentyloxy-2,2,5-trimethyl-2H-chromen

4,6 g (20 mmol) 7-Cyclopentyloxy-5-methyl-cumarin werden in 70 ml absolutem Ether gelöst und zu 100 ml einer Lösung von Methylmagnesiumiodid (1,5 g Magnesium und 8,5 g Methyliodid) in Ether unter Rühren zugefügt, worauf 5 h am Sieden gehalten wird. Der Ansatz wird über Nacht stehengelassen und mit 200 ml einer 5 %igen Ammoniumchloridlösung zersetzt. Nach üblicher Aufarbeitung und Reinigung durch Säulenchromatographie (Kieselgel-60 ; Hexan/Ether 9 : 1) erhält man ein farbloses Öl. Ausbeute : 3,8 g (74 %).

## Beispiel 3

7-s-Butyloxy-2,2-dimethyl-5-methoxy-2H-chromen

5,8 g (22 mmol) 5-s-Butyloxy-3-methoxy-2-prenylphenol werden in 100 ml Dichlormethan gelöst, mit 5 g (22 mmol) N-Iodsuccinimid versetzt und 1 h bei 30 °C gerührt. Der Ansatz wird dann mit 200 ml Wasser verdünnt ; die organische Phase wird abgetrennt, mit 5-%iger Natriumthiosulfatlösung gewaschen, getrocknet und eingeengt. Der Rückstand wird in 100 ml einer 10-%igen Lösung von Natriumhydroxid in Methanol gelöst und 2 h auf 50 °C erwärmt. Danach wird mit Wasser verdünnt und mit Ether extrahiert. Die organische Phase wird mit 1-%iger Salzsäure und Wasser gewaschen, worauf das Lösungsmittel abgetrennt und das Produkt wie in Beispiel 1 gereinigt wird. Das Produkt ist ein hellgelbes Öl. Ausbeute : 5,5 g (95 %).

## Beispiel 4

7-Ethoxy-2,2-dimethyl-5-methyl-2H-chromen

Eine Suspension aus 30 mmol 3-Ethoxy-5-methylphenol, 60 mmol 3-Chlor-3-methyl-but-1-in, 5 g Kaliumcarbonat, 8 g Kaliumiodid und 50 ml absolutem Aceton wird 20 Stunden unter Rühren gekocht. Das anorganische Salz wird dann abgesaugt ; der Rückstand wird nach Einengen des Filtrats 8 Stunden in 100 ml N,N-Dimethylanilin gekocht. Der Ansatz wird wie üblich aufgearbeitet und das Produkt analog Beispiel 2 gereinigt.

## Beispiel 5

7-Isobutoxy-2,2-dimethyl-5-methoxy-2H-chromen

3,9 g (20 mmol) 5-Isobutoxy-3-methoxyphenol und 2,8 g (20 mmol) 1,3-Dichlor-3-methylbutan werden in Gegenwart von 0,26 g (1 mmol) Bis-acetylacetonato-nickel 8 h auf 125 °C erwärmt. Der Ansatz wird dann abgekühlt und mit 25 ml Hexan verdünnt. Nach Absaugen des Katalysators und Abtrennung

des Lösungsmittels wird der Rückstand in 40 ml absolutem Benzol aufgenommen, mit 0,8 g (3,5 mmol) DDQ (Dichlordicyanobenzochinon) vermischt und 60 h am Sieden gehalten. Nach Absaugen und Einengen der Lösung wird der Rückstand analog Beispiel 1 gereinigt. Das Produkt ist ein farbloses Öl. Ausbeute : 3,6 g (69 %).

Beispiel 6

7-Cyclopentyloxy-2,2-dimethyl-5-methoxy-2H-chromen

Zu einer Lösung von 2 mmol Lithiumdiisopropylamid in 50 ml absolutem Tetrahydrofuran wird bei — 30 °C eine Lösung von 8,4 mmol 4-Cyclopentyl-2-hydroxy-6-methoxy-acetophenon in 10 ml Tetrahydrofuran zugetropft und 1 h gerührt. Der Ansatz wird auf — 40 °C abgekühlt, dann mit 10 mmol Aceton vermischt und 2 h gerührt. Danach wird mit Ether verdünnt und angesäuert ; die organische Phase wird abgetrennt, worauf das Lösungsmittel entfernt und der Rückstand in 100 ml Methanol gelöst in Gegenwart von 10 ml konz. Salzsäure 7 h gekocht wird. Anschließend wird auf 200 g Eis gegossen ; die erhaltenen Kristalle werden abgesaugt, getrocknet und analog Beispiel 1 aufgearbeitet. Das Produkt wird analog Beispiel 2 gereinigt.

In analoger Weise sind die in Tabelle 1 aufgeführten Verbindungen zugänglich.

(Siehe Tabellen Seite 6 ff.)

Untersuchung der insektwachstumshemmenden und nematociden Wirkung

Testtiere :

Insekten :

Kohlraupe (Pieris brassicae)

Senfblattkäfer (Colaphellus sophiae)

Stubenfliege (Musca domestica)

Fadenwürmer (Caenorhabditis elegans).

Die 50-EC-Formulierungen der erfindungsgemäßen Wirkstoffe besaßen folgende Zusammensetzung :

| | |
|---|---|
| Wirkstoff | 500 g |
| Arylan C.A. | 64,2 g |
| Lubrol N 13 | 40,0 g |
| Aromasol | ad 1 000,0 ml. |

Die einzelnen Versuche an den Testtieren wurden wie folgt durchgeführt :

Kohlraupe

Es wurde aus den 50-EC-Formulierungen eine Reihe von Lösungen in Wasser mit bestimmter Konzentrationsabstufung hergestellt. Kohlpflanzen wurden mit diesen Lösungen tropfnass besprüht. Nach dem Trocknen des Sprühbelags wurden die Pflanzen mit 20 Larven im II. Stadium belegt und unter langer Beleuchtung (18 h hell, 6 h dunkel) gehalten. Abgefressene Pflanzen wurden erforderlichenfalls durch gleich behandelte Pflanzen ersetzt. Es wurden die Abtötung, das Wachstum der Larven sowie eventuelle morphologische Veränderungen an den Puppen und adulten Kohlraupen bestimmt.

Senfblattkäfer

Senfpflanzen im Alter von zwei Wochen wurden mit einer Zusammensetzung aus 0,1 g Wirkstoff, 0,5 ml Dimethylsulfoxid, 10 µl 50-EC-Zusatzstoffen und 10 ml Wasser besprüht. Nach dem Trocknen des Sprühbelags wurden die Pflanzen mit 20 Larven im II. Stadium belegt. Die Pflanzen wurden alle zwei Tage durch gleich behandelte Pflanzen ersetzt. Es wurde die Anzahl der aus den Puppen kriechenden Imagines bestimmt.

Stubenfliege

Fliegenlarven wurden in 30 × 100 mm großen Glasgefäßen auf einem Nährboden aus 2 ml Milch, 2 ml Wasser, 2 g Weizenkleie und 0,1 ml einer gesättigten Lösung von Nipagin in Alkohol gehalten. Die Wirkstoffe wurden in einer Konzentration von 0,1 % in Milch gelöst, was in Bezug auf den Nährboden einer Konzentration von 0,05 % entspricht. Nach Zumischung der Wirkstoffe wurden 25 Larven im I. Stadium zugegeben, worauf die Gefäße mit einem Watteverschluß versehen wurden. Nach der Verpuppung wurden die Puppen bis zum Ausschlüpfen in Petrischalen von 25 mm Durchmesser gehalten. Es wurde die Menge der ausgeschlüpften Fliegen bestimmt.

Tabelle 1

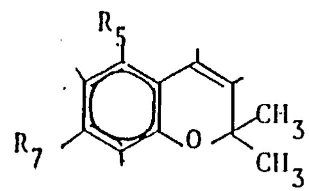

| Verbindungen Nr. | $R_5$ | $R_7$ | Beispiel Nr. | Ausbeute % | NMR |
|---|---|---|---|---|---|
| 1 | Me | Et-O | 4 | 75 | 1,32 (3H; t; J = 6 Hz); 1,36 (6H;s); 2,2 (3H; s); 3,9 (2H; q, J = 6 Hz); 5,4 (1H; d; J = 10 Hz); 6,16 (2H; m); 6,36 (1H; d; J = 10 Hz) |
| 2 | Me | n-Pr-O | 4 | 71 | 0,96 (3H; t; J = 6 Hz); 1,36 (6H; s); 1,7 (2H; m); 2,16 (3H; s); 3,8 (2H; t, J = 6 Hz); 5,38 (1H; d; J = 10 Hz); 6,18 (2H; m); 6,36 (1H; d; J = 10 Hz) |
| 3 | Me | i-Pr-O | 4 | 69 | 1,24 (6H; d; J = 6 Hz); 1,36 (6H; s); (2,16 (3H; s); 4,4 (1H; m); 5,38 (1H; d; J = 10 Hz); 6,16 (2H; m); 6,34 (1H; d; J = 10 Hz) |

Tabelle 1 (Fortsetzung)

| Verbindungen Nr. | $R_5$ | $R_7$ | Beispiel Nr. | Ausbeute % | NMR |
|---|---|---|---|---|---|
| 4 | Me | s-Bu-O | 4 | 81 | 0,92 (3H; t; J = 8 Hz); 1,22 (3H; d; J = 4 Hz); 1,34 (6H; s); 1,6 (2H, m); 2,18 (3H, s); 4,2 (1H; m); 5,4 (1H; d; J = 10 Hz); 6,16 (2H, m); 6,36 (1H; d; J = 10 Hz) |
| 5 | Me | i-Bu-O | 4 | 73 | 0,96 (6H; d; J = 7 Hz); 1,35 (6H; s); 2,0 (1H; s); 2,18 (3H; s); 3,6 (2H; d; J = 7 Hz); 5,4 (1H; d; J = 10 Hz); 6,2 (2H; m); 6,36 (1H; d; J = 10 Hz) |
| 6 | Me | c-Pentyl-O | 2 | 74 | 1,36 (6H; s); 1,8 (8H, m); 2,16 (3H; s): 4,6 (1H, m); 5,36 (1H; d; J = 10 Hz); 6,16 (2H; m); 6,36 (1H; d; J = 10 Hz) |
| 7 | Me | $CH\equiv C-CH_2O$ | 1 | 79 | 1,36 (6H; s); 2,2 (3H; s); 2,44 (1H; t; J = 2 Hz); 4,54 (2H; d; J = 2 Hz); 5,44 (1H; d; J = 10 Hz); 6,24 (2H; m); 6,36 (1H; d; J = 10 Hz) |
| 8 | Me-O | Et-O | 5 | 57 | 1,32 (3H; t; J = 7 Hz); 1,36 (6H; s); 3,68 (3H; s); 3,9 (2H; q; J = 7 Hz); 5,3 (1H; d; J = 10 Hz); 5,92 (2H; m); 6,51 (1H; d; J = 10 Hz) |
| 9 | Me-O | n-Pr-O | 3 | 86 | 0,98 (3H; t; J = 6 Hz); 1,35 (6H; s); 1,7 (2H; m); 3,7 (3H; s); 3,8 (2H;t; J = 7 Hz); 5,32 (1H; d; J = 10 Hz); 5,93 (2H; m); 6,52 (d; J = 10 Hz) |
| 10 | Me-O | i-Pr-O | 3 | 79 | 1,28 (6H; d; J = 8 Hz); 1,36 (6H; s); 3,7 (3H; s); 4,44 (1H; m); 5.32 (1H; d, J = 10 Hz); 5,92 (2H; m); 6,52 (1H; d; J = 10 Hz) |
| 11 | Me-O | s-Bu-O | 3 | 95 | 0,93 (3H; t; J = 7 Hz); 1,24 (3H; d; J = 7 Hz); 1,36 (6H; s); 1,6 (m); 3,7 (3H; s); 4,2 (1H; m); 5,31 (1H; d; J = 10 Hz); 5,92 (2H; 6,51 (1H; d; J = 10 Hz) |

Tabelle 1 (Fortsetzung)

| Verbindungen Nr. | $R_5$ | $R_7$ | Beispiel Nr. | Ausbeute % | NMR |
|---|---|---|---|---|---|
| 12 | Me-O | i-Bu-O | 13 | 69 | 0,96 (6H; d; J = 6 Hz); 1,36 (6H; s); 2,0 (1H; m); 3,62 (2H; d; J = 6 Hz); 3,7 (3H; s); 5,32 (1H; d; J = 10 Hz); 5,94 (2H; m); 6,5 (1H; d; J = 10 Hz) |
| 13 | Me-O | c-Pentyl-O | 6 | 53 | |

Caenorhabditis elegans

Auf bakterienfreie NGM-Agarplatten wurden 0,5 ml Wirkstofflösung in Aceton aufgetragen ; nach dem Trocknen des Lösungsmittels wurden die Agarplatten mit 25-30 jungen Adulten belegt. Nach 24 h wurde die Anzahl der lebenden und toten Tiere bestimmt.

Die erhaltenen Ergebnisse sind in den Tabellen 2 bis 5 angegeben.

Tabelle 2

Wirksamkeit gegenüber Kohlraupen

| Wirkstoff | Mortalität der Larven (%) | Überlebensrate (%) Puppen | Überlebensrate (%) Adulte |
|---|---|---|---|
| P1 | 41 | 59 | 50 |
| Verbindung 1 | 81 | 19 | 19 |
| Verbindung 4 | 11 | 19 | 14 |
| Verbindung 5 | 100 | - | - |
| Verbindung 6 | 100 | - | - |
| Verbindung 7 | 83 | 17 | 17 |
| Verbindung 9 | 88 | 12 | 12 |
| Verbindung 10 | 87 | 13 | 9 |
| Verbindung 11 | 100 | - | - |
| Verbindung 12 | 100 | - | - |
| Kontrolle+ | 12 | 88 | 88 |

+ = 50-EC-Formulierung ohne Wirkstoff

Wirkstoff P1 in einer Konzentration von 0,01 %, die anderen Wirkstoffe von 0,1 % in 50-EC-Formulierung.

Tabelle 3

Wirksamkeit gegenüber Senfblattkäfern

| Wirkstoff | Konzentration (%) | Überlebensrate Adulte (%) |
|---|---|---|
| Verbindung 5 | 1 | 27 |
| Kontrolle | 0 | 100 |

(Siehe Tabelle 4 Seite 10 f.)

Tabelle 4

Wirksamkeit gegenüber Stubenfliegen

| Wirkstoff | Überlebensrate (%) Puppen | Überlebensrate (%) Adulte |
|---|---|---|
| P1 | 100 | 33,3 |
| Verbindung 1 | 40 | 14,8 |
| Verbindung 3 | 50,9 | 27,6 |
| Verbindung 4 | 25,4 | 12,5 |
| Verbindung 5 | 45,7 | 25,5 |
| Verbindung 6 | 52,7 | 31,8 |
| Verbindung 7 | 50,9 | 10,6 |
| Verbindung 9 | 9,1 | 6,4 |
| Verbindung 12 | 0 | 0 |
| Kontrolle | 100 | 100 |

Tabelle 5

Wirksamkeit gegenüber Caenorhabditis elegans

| Wirkstoff | Konzentration (µg/ml) | Mortalität (%) | Nachkommen | Bemerkung |
|---|---|---|---|---|
| P1 | 200 | 44 | reduziert | |
| | 400 | 98 | keine | |
| P2 | 200 | 100 | keine | |
| | 400 | 100 | keine | |
| P3 | 200 | 70 | einige | |
| | 400 | 90 | keine | |
| Verbindung 7 | 200 | 20 | einige | lahm |
| | 400 | 30 | keine | lahm |
| Kontrolle | - | 0 | normal | |
| 10% Aceton | - | 0 | normal | |

**Beschreibung** (für den Vertragsstaat AT)

Die Erfindung betrifft ein Verfahren zur Herstellung neuer Chromenderivate der Formel I

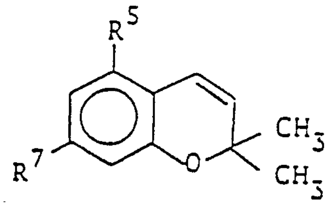

(I)

worin bedeuten :

$R^5$ Methyl oder Methoxy und

$R^7$ $C_{2-4}$-Alkoxy, $C_{3-5}$-Cycloalkoxy oder Propargyloxy,

sowie ihre Verwendung als Wirkstoffe in Schädlingsbekämpfungsmitteln.

Die erfindungsgemäßen eingesetzten Verbindungen sind Analoga der Naturstoffe Precocen-1 (P1) und Precocen-2 (P2). Die biologischen Wirkungen der Verbindungen P1 und P2 sind in der Literatur beschrieben (Bowers, W.S., et al., Science 193 (1976) 542 ; Brooks, G.T., et al., Nature 281 (1979) 570-572). Aufgrund ihrer biologischen Wirkung auf Insekten können diese Verbindungen als neue, umweltschonende Schädlingsbekämpfungsmittel verwendet werden. Die biologische Wirksamkeit, der Wirkungsmechanismus und der Metabolismus der Precocene sind in zahlreichen Publikationen beschrieben, wonach ihre Wirkung auf der Schädigung der Corpora allata der Insekten, welche die Juvenilhormone bilden, beruht. Ferner wurde bestimmt, wie die biologische Wirkung vom 2H-Chromenring abhängig ist. Bei den Untersuchungen wurde festgestellt, daß die Art und Stärke der Wirksamkeit der Precocene stark von der Art der Schädlinge, von den Testmethoden und, hinsichtlich der chemischen Struktur, von der Art und Anzahl sowie Stellung der Substituenten des aromatischen Rings, der Stärke der Doppelbindung im Pyranring sowie von den elektronischen und sterischen Parametern des ganzen Moleküls abhängig ist (Bowers, W.S., Martinez, P.R., Science 197 (1977) 1369 ; Schooneveld, H., Experientia 35 (1979) 363 ; Bowers, W.S., Pontif. Acad. Sci. Scr. Varia 41 (1976) 129 ; Brooks, C.T., et al., Nature 281 (1979) 570 ; Ohta, T., Kagaku to Seibutsu 17 (2) (1979) 92 ; Ohta, T., Konchu no Seiri to Kagaku (1979) 63 ; Matolcsy, G., et al., Z. Naturforsch. 35b (1980) 1449 ; Brooks, G.T., et al., Proc. Br. Crop. Prot. Conf. Pests. Dis. 1 (1979) 273 ; Pratt, G.E., et al., Nature 284 (1980) 320 ; Soderlund, D.M., et al., J. Agr. Food Chem. 28 (4) (1980) 724 ; Schooley, D.A., et al., Pestic. Biochem. Physiol. 13 (2) (1980) 95 ; Ottridge, A.P., et al., Pestic. Sci. 12 (3) (1981) 245 ; Sci. Papers of the Inst. of Org. and Phys. Chem. of Wroclaw Techn. Univ. 22 (7) (1981) 309-424 ; Pratt, G.E., Brooks, G.T. ed., Juvenile Hormone Biochemistry, Elsevier/North-Holland Biomed Press (1981) 311-437.

Im Rahmen dieser Untersuchungen wurden Derivate, wie 6-Methoxy-7-ethoxy-2,2-dimethyl-2H-chromen (P3) und 6-Methoxy-7-isopropoxy-2,2-dimethyl-2H-chromen, synthetisiert, die deutliche größere Aktivität zeigen als die in der Natur vorkommenden Precocene P1 und P2. Die Anzahl der bisher synthetisierten Precocenanaloga ist jedoch gering.

Es wurden auch mehrere, praktisch voneinander unabhängige Synthesen zur Herstellung von 2H-Chromenen angegeben (Chromenes, Chromanones and Chromones, G.P. Ellis ed., John Wiley and Sons, London (1977) 43-66 ; DE-PS 2 639 671, US-PS 4 162 326, JP-PSen 73121/79, 15411/80, 40637/80 und 43039/80, ES-PSen 496 301 und 496 302).

Eine bessere Kenntnis des Zusammenhangs zwischen Struktur und Wirkung würde zu einer Reihe von Verbindungen mit hoher Wirkung führen. Bis jetzt wurde jedoch die Anti-Juvenilhormon-Aktivität bei höchstens 30 bis 50 Verbindungen und nur bei 1 bis 2 Insektenarten bestimmt. Über ihre insecticide Wirkung, die sterilisierende, oocide sowie diapauseninduzierende Wirkung liegen fast keine Informationen vor.

Der Erfindung liegt entsprechend die Aufgabe zugrunde, ein Verfahren zur Herstellung neuer Chromenderivate, die Precocenanaloga darstellen und hohe insecticide sowie nematocide Wirkung besitzen, sowie entsprechende Schädlingsbekämpfungsmittel anzugeben.

Die Aufgabe wird anspruchsgemäß gelöst. Vorteilhafte Ausführungsformen sind Gegenstände der abhängigen Ansprüche.

Das erfindungsgemäße Verfahren zur Herstellung von Chromenderivaten der Formel I

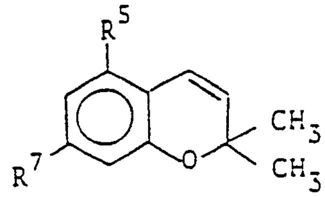

(I)

worin bedeuten :

$R^5$ Methyl oder Methoxy und

$R^7$ $C_{2-4}$-Alkoxy, $C_{3-5}$-Cycloalkoxy oder Propargyloxy,

ist gekennzeichnet durch Cyclisierung von Arylpropargylethern der Formel IV

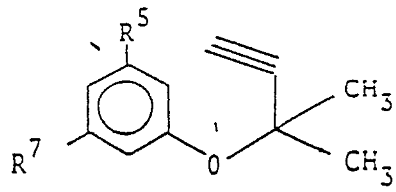

(IV)

mit $R^5$ und $R^7$ wie oben.

Die erfindungsgemäßen Schädlingsbekämpfungsmittel sind gekennzeichnet durch einen Gehalt an Chromenderivaten der Formel I

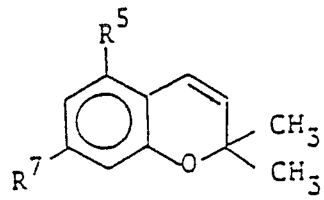

(I)

worin bedeuten :

$R^5$ Methyl oder Methoxy und

$R^7$ $C_{2-4}$-Alkoxy, $C_{3-5}$-Cycloalkoxy oder Propargyloxy,

als Wirkstoffen neben üblichen festen Trägerstoffen und/oder flüssigen Verdünnungsmitteln und gegebenenfalls grenzflächenaktiven Stoffen.

Sie enthalten bevorzugt Chromenderivate der Formel II

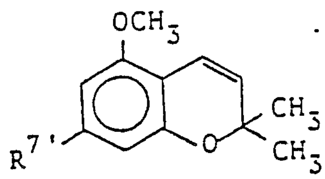

(II)

worin $R^{7'}$ n-Propoxy, i-Propoxy, s-Butoxy oder i-Butoxy bedeutet, oder Chromenderivate der Formel III

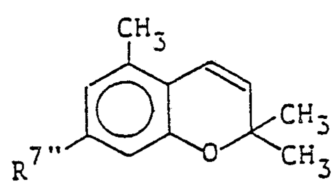

(III)

worin $R^{7''}$ Ethoxy, i-Propoxy, s-Butoxy oder n-Cyclopentyloxy bedeutet.

Die Arylpropargylether der Formel IV, aus denen die erfindungsgemäß eingesetzten Chromenderivate der Formeln I, II und III durch Cyclisierung zugänglich sind, werden durch Umsetzung von Phenolen der Formel V

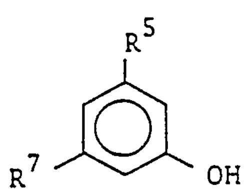

(V)

mit $R^5$ und $R^7$ wie oben mit Acetylenderivaten der Formel VI

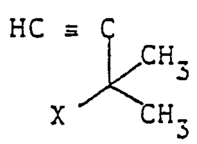

(VI)

mit X = Halogen hergestellt.

Diese Reaktion ist an sich bekannt (H. Hlubucek, E. Ritchie, W.C. Taylor, Aust. J. Chem. 11 (1971) 2347 ; R. Chenevert et al., Experientia 36 (1980) 379).

Das erfindungsgemäße Verfahren kann vorzugsweise wie folgt durchgeführt werden : Die Phenole der Formel V werden mit den Acetylenderivaten der Formel VI in Gegenwart von Basen, in Gegenwart von Alkaliiodiden als Katalysatoren sowie in Gegenwart von bipolaren aprotischen Lösungsmitteln zu den Arylpropargylethern der Formel VI umgesetzt. Ihre Cyclisierung erfolgt mit N,N-Dialkylarylaminen.

Die erfindungsgemäßen Schädlingsbekämpfungsmittel enthalten als Wirkstoffe Verbindungen der Formel I sowie übliche feste Trägerstoffe und/oder flüssige Verdünnungsmittel, wie Füllstoffe, Netzmittel, Dispergiermittel und/oder Emulgiermittel und/oder grenzflächenaktive Stoffe.

Die Erfindung wird im folgenden anhand von Beispielen näher erläutert.

Die Reinheit der erhaltenen Verbindungen wurde durch Dünnschichtchromatographie und Gaschromatographie, ihre Struktur durch IR-, NMR- und Massenspektrometrie überprüft.

Beispiel für die Angabe von NMR Spektren :

1,45 (3H ; t ; J = 5Hz) : darin bedeuten :
1,45 - die chemische Verschiebung
3H - die Anzahl der Protonen
t - die Multiplizität
J - die Kopplungskonstante.

Bezeichnung der Multipletts :
s - Singulett
d - Dublett
t - Triplett
q - Quartett
m - größeres Multiplett
b - breite Spitze

## Beispiel 1

2,2-Dimethyl-5-methyl-7-propargyloxy-2H-chromen

20 mmol 2,2-Dimethyl-5-methyl-7-propargyloxy-chromanon werden in 100 ml eines Gemischs von Tetrahydrofuran/Wasser (1 : 1) gelöst und mit 5,3 g (30 mmol) Palladiumchlorid und 8,31 g (220 mmol) Natriumtetrahydroborat bei 0 °C 3 Stunden gerührt. Danach wird abgesaugt, das Filtrat dreimal mit je 100 ml Ether extrahiert und das Lösungsmittel entfernt. Der Rückstand wird in 300 ml Dichlormethan gelöst und mit 15 ml Pyridin und 2 g Phosphoroxychlorid 1 Stunde auf 35 °C erwärmt. Der Ansatz wird dann auf 100 ml Wasser gegossen und mit 5 % Salzsäure und mit Wasser gewaschen. Die organische Phase wird getrocknet ; nach Abtrennung des Lösungsmittels wird der Rückstand aus Methanol umkristallisiert. Ausbeute : 3,8 g (60 %), Fp. : 80-85 °C.

## Beispiel 2

7-Cyclopentyloxy-2,2,5-trimethyl-2H-chromen

4,6 g (20 mmol) 7-Cyclopentyloxy-5-methyl-cumarin werden in 70 ml absolutem Ether gelöst und zu 100 ml einer Lösung von Methylmagnesiumiodid (1,5 g Magnesium und 8,5 g Methyliodid) in Ether unter Rühren zugefügt, worauf 5 h am Sieden gehalten wird. Der Ansatz wird über Nacht stehengelassen und mit 200 ml einer 5 %igen Ammoniumchloridlösung zersetzt. Nach üblicher Aufarbeitung und Reinigung durch Säulenchromatographie (Kieselgel-60 ; Hexan/Ether 9 : 1) erhält man ein farbloses Öl. Ausbeute : 3,8 g (74 %).

## Beispiel 3

7-s-Butyloxy-2,2-dimethyl-5-methoxy-2H-chromen

5,8 g (22 mmol) 5-s-Butyloxy-3-methoxy-2-prenylphenol werden in 100 ml Dichlormethan gelöst, mit 5 g (22 mmol) N-Iodsuccinimid versetzt und 1 h bei 30 °C gerührt. Der Ansatz wird dann mit 200 ml Wasser verdünnt ; die organische Phase wird abgetrennt, mit 5-%iger Natriumthiosulfatlösung gewaschen, getrocknet und eingeengt. Der Rückstand wird in 100 ml einer 10-%igen Lösung von Natriumhydroxid in Methanol gelöst und 2 h auf 50 °C erwärmt. Danach wird mit Wasser verdünnt und mit Ether extrahiert. Die organische Phase wird mit 1-%iger Salzsäure und Wasser gewaschen, worauf das Lösungsmittel abgetrennt und das Produkt wie in Beispiel 1 gereinigt wird. Das Produkt ist ein hellgelbes Öl. Ausbeute : 5,5 g (95 %).

Beispiel 4

7-Ethoxy-2,2-dimethyl-5-methyl-2H-chromen

Eine Suspension aus 30 mmol 3-Ethoxy-5-methylphenol, 60 mmol 3-Chlor-3-methyl-but-1-in, 5 g Kaliumcarbonat, 8 g Kaliumiodid und 50 ml absolutem Aceton wird 20 Stunden unter Rühren gekocht. Das anorganische Salz wird dann abgesaugt ; der Rückstand wird nach Einengen des Filtrats 8 Stunden in 100 ml N,N-Dimethylanilin gekocht. Der Ansatz wird wie üblich aufgearbeitet und das Produkt analog Beispiel 2 gereinigt.

Beispiel 5

7-Isobutoxy-2,2-dimethyl-5-methoxy-2H-chromen

3,9 g (20 mmol) 5-Isobutoxy-3-methoxyphenol und 2,8 g (20 mmol) 1,3-Dichlor-3-methylbutan werden in Gegenwart von 0,26 g (1 mmol) Bis-acetylacetonato-nickel 8 h auf 125 °C erwärmt. Der Ansatz wird dann abgekühlt und mit 25 ml Hexan verdünnt. Nach Absaugen des Katalysators und Abtrennung des Lösungsmittels wird der Rückstand in 40 ml absolutem Benzol aufgenommen, mit 0,8 g (3,5 mmol) DDQ (Dichlordicyanobenzochinon) vermischt und 60 h am Sieden gehalten. Nach Absaugen und Einengen der Lösung wird der Rückstand analog Beispiel 1 gereinigt. Das Produkt ist ein farbloses Öl. Ausbeute : 3,6 g (69 %).

Beispiel 6

7-Cyclopentyloxy-2,2-dimethyl-5-methoxy-2H-chromen

Zu einer Lösung von 2 mmol Lithiumdiisopropylamid in 50 ml absolutem Tetrahydrofuran wird bei — 30 °C eine Lösung von 8,4 mmol 4-Cyclopentyl-2-hydroxy-6-methoxy-acetophenon in 10 ml Tetrahydrofuran zugetropft und 1 h gerührt. Der Ansatz wird auf — 40 °C abgekühlt, dann mit 10 mmol Aceton vermischt und 2 h gerührt. Danach wird mit Ether verdünnt und angesäuert ; die organische Phase wird abgetrennt, worauf das Lösungsmittel entfernt und der Rückstand in 100 ml Methanol gelöst in Gegenwart von 10 ml konz. Salzsäure 7 h gekocht wird. Anschließend wird auf 200 g Eis gegossen ; die erhaltenen Kristalle werden abgesaugt, getrocknet und analog Beispiel 1 aufgearbeitet. Das Produkt wird analog Beispiel 2 gereinigt.

In analoger Weise sind die in Tabelle 1 aufgeführten Verbindungen zugänglich.

(Siehe Tabellen Seite 15 ff.)

Untersuchung der insektwachstumshemmenden und nematociden Wirkung
Testtiere :
Insekten :
Kohlraupe (Pieris brassicae)
Senfblattkäfer (Colaphellus sophiae)
Stubenfliege (Musca domestica)
Fadenwürmer (Caenorhabditis elegans).

Die 50-EC-Formulierungen der erfindungsgemäßen eingesetzten Wirkstoffe besaßen folgende Zusammensetzung :

| | |
|---|---|
| Wirkstoff | 500 g |
| Arylan C. A. | 64,2 g |
| Lubrol N 13 | 40,0 g |
| Aromasol | ad 1 000,0 ml. |

Die einzelnen Versuche an den Testtieren wurden wie folgt durchgeführt :

Kohlraupe

Es wurde aus den 50-EC-Formulierungen eine Reihe von Lösungen in Wasser mit bestimmter Konzentrationsabstufung hergestellt. Kohlpflanzen wurden mit diesen Lösungen tropfnass besprüht. Nach dem Trocknen des Sprühbelags wurden die Pflanzen mit 20 Larven im II. Stadium belegt und unter langer Beleuchtung (18 h hell, 6 h dunkel) gehalten. Abgefressene Pflanzen wurden erforderlichenfalls durch gleich behandelte Pflanzen ersetzt. Es wurden die Abtötung, das Wachstum der Larven sowie eventuelle morphologische Veränderungen an den Puppen und adulten Kohlraupen bestimmt.

Tabelle 1

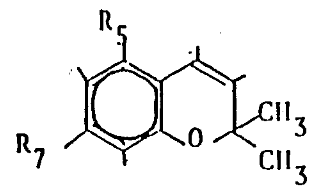

| Verbindungen Nr. | $R_5$ | $R_7$ | Beispiel Nr. | Ausbeute % | NMR |
|---|---|---|---|---|---|
| 1 | Me | Et-O | 4 | 75 | 1,32 (3H; t; J = 6 Hz); 1,36 (6H;s); 2,2 (3H; s); 3,9 (2H; q, J = 6 Hz); 5,4 (1H; d; J = 10 Hz); 6,16 (2H; m); 6,36 (1H; d; J = 10 Hz) |
| 2 | Me | n-Pr-O | 4 | 71 | 0,96 (3H; t; J = 6 Hz); 1,36 (6H; s); 1,7 (2H; m); 2,16 (3H; s); 3,8 (2H; t, J = 6 Hz); 5,38 (1H; d; J = 10 Hz); 6,18 (2H; m); 6,36 (1H; d; J = 10 Hz) |
| 3 | Me | i-Pr-O | 4 | 69 | 1,24 (6H; d; J = 6 Hz); 1,36 (6H; s); (2,16 (3H; s); 4,4 (1H; m); 5,38 (1H; d; J = 10 Hz); 6,16 (2H; m); 6,34 (1H; d; J = 10 Hz) |

Tabelle 1 (Fortsetzung)

| Verbindungen Nr. | $R_5$ | $R_7$ | Beispiel Nr. | Ausbeute % | NMR |
|---|---|---|---|---|---|
| 4 | Me | s-Bu-O | 4 | 81 | 0,92 (3H; t; J = 8 Hz); 1,22 (3H; d; J = 4 Hz); 1,34 (6H; s); 1,6 (2H, m); 2,18 (3H, s); 4,2 (1H; m); 5,4 (1H; d; J = 10 Hz); 6,16 (2H, m); 6,36 (1H; d; J = 10 Hz) |
| 5 | Me | i-Bu-O | 4 | 73 | 0,96 (6H; d; J = 7 Hz); 1,35 (6H; s); 2,0 (1H; s); 2,18 (3H; s); 3,6 (2H; d; J = 7 Hz); 5,4 (1H; d; J = 10 Hz); 6,2 (2H; m); 6,36 (1H; d; J = 10 Hz) |
| 6 | Me | c-Pentyl-O | 2 | 74 | 1,36 (6H; s); 1,8 (8H, m); 2,16 (3H; s): 4,6 (1H, m); 5,36 (1H; d; J = 10 Hz); 6,16 (2H; m); 6,36 (1H; d; J = 10 Hz) |
| 7 | Me | CH≡C-$CH_2$O | 1 | 79 | 1,36 (6H; s); 2,2 (3H; s); 2,44 (1H; t; J = 2 Hz); 4,54 (2H; d; J = 2 Hz); 5,44 (1H; d; J = 10 Hz); 6,24 (2H; m); 6,36 (1H; d; J = 10 Hz) |
| 8 | Me-O | Et-O | 5 | 57 | 1,32 (3H; t; J = 7 Hz); 1,36 (6H; s); 3,68 (3H; s); 3,9 (2H; q; J = 7 Hz); 5,3 (1H; d; J = 10 Hz); 5,92 (2H; m); 6,51 (1H; d; J = 10 Hz) |
| 9 | Me-O | n-Pr-O | 3 | 86 | 0,98 (3H; t; J = 6 Hz); 1,35 (6H; s); 1,7 (2H; m); 3,7 (3H; s); 3,8 (2H;t; J = 7 Hz); 5,32 (1H; d; J = 10 Hz); 5,93 (2H; m); 6,52 (d; J = 10 Hz) |
| 10 | Me-O | i-Pr-O | 3 | 79 | 1,28 (6H; d; J = 8 Hz); 1,36 (6H; s); 3,7 (3H; s); 4,44 (1H; m); 5.32 (1H; d, J = 10 Hz); 5,92 (2H; m); 6,52 (1H; d; J = 10 Hz) |
| 11 | Me-O | s-Bu-O | 3 | 95 | 0,93 (3H; t; J = 7 Hz); 1,24 (3H; d; J = 7 Hz); 1,36 (6H; s); 1,6 (m); 3,7 (3H; s); 4,2 (1H; m); 5,31 (1H; d; J = 10 Hz); 5,92 (2H; 6,51 (1H; d; J = 10 Hz) |

Tabelle 1 (Fortsetzung)

| Verbindungen Nr. | $R_5$ | $R_7$ | Beispiel Nr. | Ausbeute % | NMR |
|---|---|---|---|---|---|
| 12 | Me-O | i-Bu-O | 13 | 69 | 0,96 (6H; d; J = 6 Hz); 1,36 (6H; s); 2,0 (1H; m); 3,62 (2H; d; J = 6 Hz); 3,7 (3H; s); 5,32 (1H; d; J = 10 Hz); 5,94 (2H; m); 6,5 (1H; d; J = 10 Hz) |
| 13 | Me-O | c-Pentyl-O | 6 | 53 | |

Senfblattkäfer

Senfpflanzen im Alter von zwei Wochen wurden mit einer Zusammensetzung aus 0,1 g Wirkstoff, 0,5 ml Dimethylsulfoxid, 10 µl 50-EC-Zusatzstoffen und 10 ml Wasser besprüht. Nach dem Trocknen des Sprühbelags wurden die Pflanzen mit 20 Larven im II. Stadium belegt. Die Pflanzen wurden alle zwei Tage durch gleich behandelte Pflanzen ersetzt. Es wurde die Anzahl der aus den Puppen kriechenden Imagines bestimmt.

Stubenfliege

Fliegenlarven wurden in 30 × 100 mm großen Glasgefäßen auf einem Nährboden aus 2 ml Milch, 2 ml Wasser, 2 g Weizenkleie und 0,1 ml einer gesättigten Lösung von Nipagin in Alkohol gehalten. Die Wirkstoffe wurden in einer Konzentration von 0,1 % in Milch gelöst, was in Bezug auf den Nährboden einer Konzentration von 0,05 % entspricht. Nach Zumischung der Wirkstoffe wurden 25 Larven im I. Stadium zugegeben, worauf die Gefäße mit einem Watteverschluß versehen wurden. Nach der Verpuppung wurden die Puppen bis zum Ausschlüpfen in Petrischalen von 25 mm Durchmesser gehalten. Es wurde die Menge der ausgeschlüpften Fliegen bestimmt.

Caenorhabditis elegans

Auf bakterienfreie NGM-Agarplatten wurden 0,5 ml Wirkstofflösung in Aceton aufgetragen ; nach dem Trocknen des Lösungsmittels wurden die Agarplatten mit 25-30 jungen Adulten belegt. Nach 24 h wurde die Anzahl der lebenden und toten Tiere bestimmt.

Die erhaltenen Ergebnisse sind in den Tabellen 2 bis 5 angegeben.

Tabelle 2

Wirksamkeit gegenüber Kohlraupen

| Wirkstoff | Mortalität der Larven (%) | Überlebensrate (%) Puppen | Überlebensrate (%) Adulte |
|---|---|---|---|
| P1 | 41 | 59 | 50 |
| Verbindung 1 | 81 | 19 | 19 |
| Verbindung 4 | 11 | 19 | 14 |
| Verbindung 5 | 100 | - | - |
| Verbindung 6 | 100 | - | - |
| Verbindung 7 | 83 | 17 | 17 |
| Verbindung 9 | 88 | 12 | 12 |
| Verbindung 10 | 87 | 13 | 9 |
| Verbindung 11 | 100 | - | - |
| Verbindung 12 | 100 | - | - |
| Kontrolle+ | 12 | 88 | 88 |

+ = 50-EC-Formulierung ohne Wirkstoff
Wirkstoff P1 in einer Konzentration von 0,01 %, die anderen Wirkstoffe von 0,1 % in 50-EC-Formulierung.

(Siehe Tabelle 3 Seite 19 f.)

Tabelle 3

Wirksamkeit gegenüber Senfblattkäfern

| Wirkstoff | Konzentration (%) | Überlebensrate Adulte (%) |
|---|---|---|
| Verbindung 5 | 1 | 27 |
| Kontrolle | 0 | 100 |

Tabelle 4

Wirksamkeit gegenüber Stubenfliegen

| Wirkstoff | Überlebensrate (%) | |
|---|---|---|
| | Puppen | Adulte |
| P1 | 100 | 33,3 |
| Verbindung 1 | 40 | 14,8 |
| Verbindung 3 | 50,9 | 27,6 |
| Verbindung 4 | 25,4 | 12,5 |
| Verbindung 5 | 45,7 | 25,5 |
| Verbindung 6 | 52,7 | 31,8 |
| Verbindung 7 | 50,9 | 10,6 |
| Verbindung 9 | 9,1 | 6,4 |
| Verbindung 12 | 0 | 0 |
| Kontrolle | 100 | 100 |

Tabelle 5

Wirksamkeit gegenüber Caenorhabditis elegans

| Wirkstoff | Konzentration (µg/ml) | Mortalität (%) | Nachkommen | Bemerkung |
|---|---|---|---|---|
| P1 | 200 | 44 | reduziert | |
| | 400 | 98 | keine | |
| P2 | 200 | 100 | keine | |
| | 400 | 100 | keine | |
| P3 | 200 | 70 | einige | |
| | 400 | 90 | keine | |

Tabelle 5 (Fortsetzung)

| Wirkstoff | Konzentration (µg/ml) | Mortalität (%) | Nachkommen | Bemerkung |
|---|---|---|---|---|
| Verbindung 7 | 200 | 20 | einige | lahm |
| | 400 | 30 | keine | lahm |
| Kontrolle | - | 0 | normal | |
| 10% Aceton | - | 0 | normal | |

**Patentansprüche** (für die Vertragsstaaten : BE, CH, DE, FR, GB, IT, LI, NL, SE)

1. Chromenderivate der Formel I

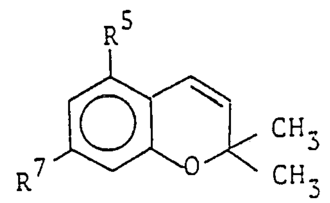

(I)

worin bedeuten :

$R^5$ Methyl oder Methoxy und

$R^7$ $C_{2-4}$-Alkoxy, $C_{3-5}$-Cycloalkoxy oder Propargyloxy.

2. Chromenderivate nach Anspruch 1 der Formel II

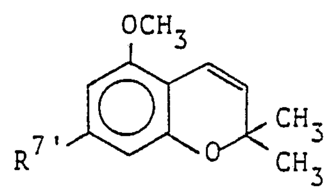

(II)

worin $R^{7'}$ n-Propoxy, i-Propoxy, s-Butoxy oder i-Butoxy bedeutet.

3. Chromenderivate nach Anspruch 1 der Formel III

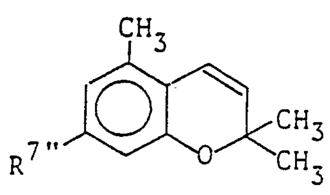

(III)

worin $R^{7''}$ Ethoxy, i-Propoxy, s-Butoxy oder n-Cyclopentyloxy bedeutet.

4. Verfahren zur Herstellung der Chromenderivate der Formel I bzw. II und III nach einem der Ansprüche 1 bis 3, gekennzeichnet durch Cyclisierung von Arylpropargylethern der Formel IV

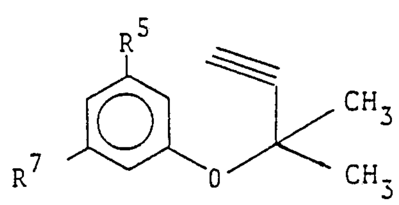

(IV)

mit $R^5$ und $R^7$ wie in Anspruch 1.

5. Schädlingsbekämpfungsmittel, gekennzeichnet durch einen Gehalt an Chromenderivaten der Formel I nach Anspruch 1 als Wirkstoffen neben üblichen festen Trägerstoffen und/oder flüssigen Verdünnungsmitteln und gegebenenfalls grenzflächenaktiven Stoffen.

6. Schälingsbekämpfungsmittel nach Anspruch 5, gekennzeichnet durch einen Gehalt an Chromenderivaten der Formel II nach Anspruch 2 als Wirkstoffen.

7. Schälingsbekämpfungsmittel nach Anspruch 5, gekennzeichnet durch einen Gehalt an Chromenderivaten der Formel III nach Anspruch 3 als Wirkstoffen.

**Patentansprüche** (für den Vertragsstaat AT)

1. Verfahren zur Herstellung von Chromenderivaten der Formel I

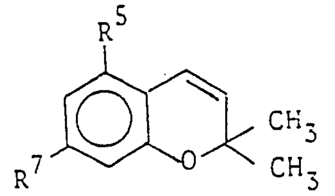

(I)

worin bedeuten :

$R^5$ Methyl oder Methoxy und

$R^7$ $C_{2-4}$-Alkoxy, $C_{3-5}$-Cycloalkoxy oder Propargyloxy,

gekennzeichnet durch Cyclisierung von Arylpropargylethern der Formel IV

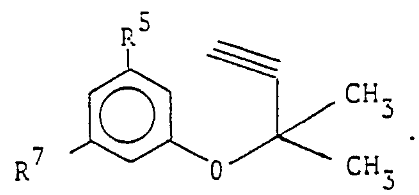

(IV)

mit $R^5$ und $R^7$ wie oben.

2. Schädlingsbekämpfungsmittel, gekennzeichnet durch einen Gehalt an Chromenderivaten der Formel I

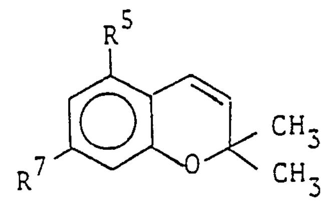

(I)

worin bedeuten :

$R^5$ Methyl oder Methoxy und

$R^7$ $C_{2-4}$-Alkoxy, $C_{3-5}$-Cycloalkoxy oder Propargyloxy,

als Wirkstoffen neben üblichen festen Trägerstoffen und/oder flüssigen Verdünnungsmitteln und gegebenenfalls grenzflächenaktiven Stoffen.

3. Schädlingsbekämpfungsmittel nach Anspruch 2, gekennzeichnet durch einen Gehalt an Chromenderivaten der Formel II

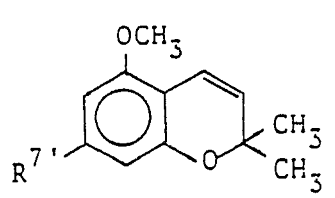

(II)

worin $R^{7'}$ n-Propoxy, i-Propoxy, s-Butoxy oder i-Butoxy bedeutet, als Wirkstoffen neben üblichen festen Trägerstoffen und/oder flüssigen Verdünnungsmitteln und gegebenenfalls grenzflächenaktiven Stoffen.

4. Schädlingsbekämpfungsmittel nach Anspruch 2, gekennzeichnet durch einen Gehalt an Chromenderivaten der Formel III

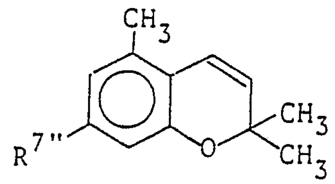

(III)

worin $R^{7''}$ Ethoxy, i-Propoxy, s-Butoxy oder n-Cyclopentyloxy bedeutet, als Wirkstoffen neben üblichen festen Trägerstoffen und/oder flüssigen Verdünnungsmitteln und gegebenenfalls grenzflächenaktiven Stoffen.

**Claims** (for the Contracting States : BE, CH, DE, FR, GB, IT, LI, NL, SE)

1. Chromene derivatives of formula I

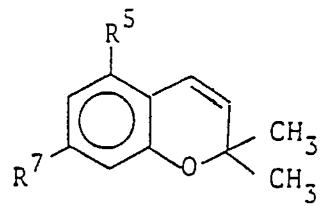

(I)

wherein :

$R^5$ represents methyl or methoxy and

$R^7$ represents $C_{2-4}$ alkoxy, $C_{3-5}$ cycloalkoxy or propargyloxy.

2. Chromene derivatives as claimed in claim 1 of formula II

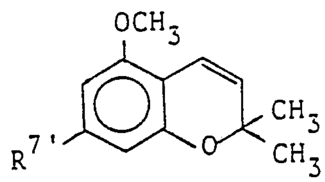

(II)

wherein $R^{7'}$ represents n-propoxy, i-propoxy, s-butoxy or i-butoxy.

3. Chromene derivatives as claimed in claim 1 of formula III

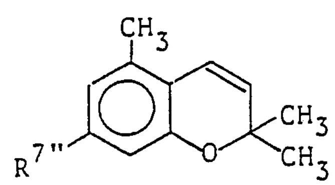

(III)

wherein $R^{7''}$ represents ethoxy, i-propoxy, s-butoxy or n-cyclopentyloxy.

4. Process for preparing the chromene derivatives of formula I or II and III as claimed in one of claims 1 to 3, characterised by cyclisation of arylpropargyl ethers of formula IV

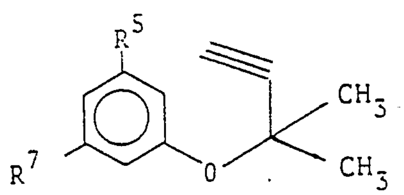

(IV)

with $R^5$ and $R^7$ as in claim 1.

5. Pesticides, characterised in that they contain chromene derivatives of formula I as claimed in claim 1 as active substances together with conventional solid carriers and/or liquid diluents and optionally interface-active substances.

6. Pesticides as claimed in claim 5, characterised in that they contain chromene derivatives of formula II as claimed in claim 2 as active substances.

7. Pesticides as claimed in claim 5, characterised in that they contain chromene derivatives of formula III as claimed in claim 3 as active substances.

**Claims** (for the Contracting State AT)

1. Process for preparing chromene derivatives of formula I

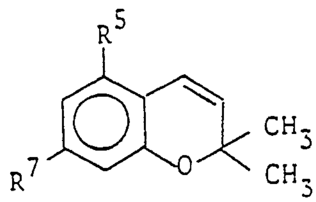

(I)

wherein
$R^5$ represents methyl or methoxy and
$R^7$ represents $C_{2\text{-}4}$ alkoxy, $C_{3\text{-}5}$ cycloalkoxy or propargyloxy,
characterised by cyclisation of arylpropargylethers of formula IV

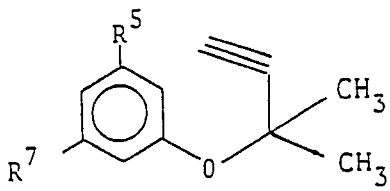

(IV)

wherein $R^5$ and $R^7$ are as above.

2. Pesticides, characterised in that they contain chromene derivatives of formula I

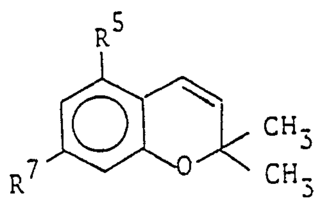

(I)

wherein
$R^5$ represents methyl or methoxy and
$R^7$ represents $C_{2\text{-}4}$ alkoxy, $C_{3\text{-}5}$ cycloalkoxy or propargyloxy,
as active substances together with conventional solid carriers and/or liquid diluents and optionally interface-active substances.

3. Pesticides as claimed in claim 2, characterised in that they contain chromene derivatives of formula II

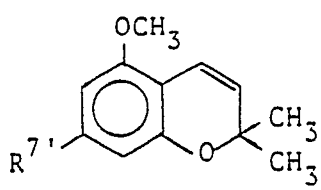

(II)

wherein $R^{7'}$ represents n-propoxy, i-propoxy, s-butoxy or i-butoxy as active substances together with conventional solid carriers and/or liquid diluents and optionally interface-active substances.

4. Pesticides as claimed in claim 2, characterised in that they contain chromene derivatives of formula III

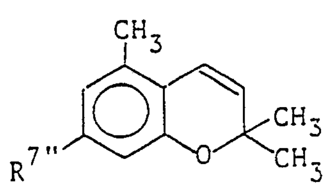

(III)

wherein $R^{7''}$ represents ethoxy, i-propoxy, s-butoxy or n-cyclopentyloxy, as active substances together with conventional solid carriers and/or liquid diluents and optionally interface-active substances.

**Revendications** (pour les Etats contractants : BE, CH, DE, FR, GB, IT, LI, NL, SE)

1. Dérivés du chromène de formule I

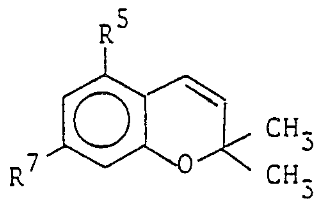

(I)

dans laquelle
$R^5$ représente un groupe méthyle ou méthoxy, et
$R^7$ représente un groupe alcoxy en C2-C4, cycloalcoxy en C3-C5 ou propargyloxy.

2. Dérivés du chromène selon la revendication 1, de formule II

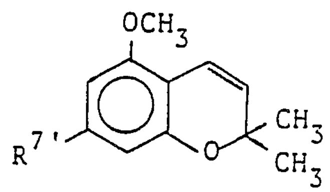

(II)

dans laquelle $R^{7'}$ représente un groupe n-propoxy, isopropoxy, sec-butoxy ou isobutoxy.

3. Dérivés du chromène selon la revendication 1, de formule III

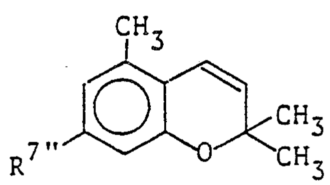

(III)

dans laquelle $R^{7''}$ représente un groupe éthoxy, isopropoxy, sec-butoxy ou n-cyclopentyloxy.

4. Procédé de préparation des dérivés du chromène de formule I ou respectivement II et III selon l'une des revendications 1 à 3, caractérisé en ce que l'on cyclise des éthers arylpropargyliques de formule IV

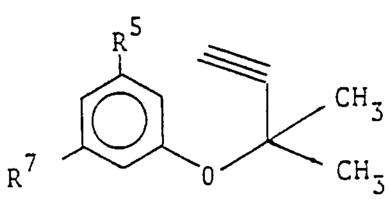

(IV)

dans laquelle $R^5$ et $R^7$ ont les significations indiquées dans la revendication 1.

5. Produit pesticide, caractérisé en ce qu'il contient des dérivés du chromène de formule I selon la revendication 1 en tant que substances actives, avec des véhicules solides et/ou des diluants liquides usuels et le cas échéant des substances tensioactives.

6. Produit pesticide selon la revendication 5, caractérisé en ce qu'il contient des dérivés du chromène de formule II selon la revendication II en tant que substances actives.

7. Produit pesticide selon la revendication 5, caractérisé en ce qu'il contient des dérivés du chromène de formule III selon la revendication 3 en tant que substances actives.

**Revendications** (pour l'Etat contractant AT)

1. Procédé pour la préparation de dérivés du chromène de formule I

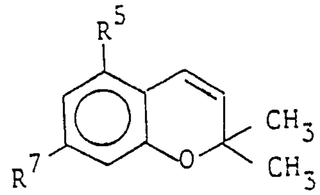

(I)

dans laquelle
$R^5$ représente un groupe méthyle ou méthoxy, et
$R^7$ représente un groupe alcoxy en C2-C4, cycloalcoxy en C3-C5 ou propargyloxy,
caractérisé en ce que l'on effectue une cyclisation des éthers arylpropargyliques de formule IV

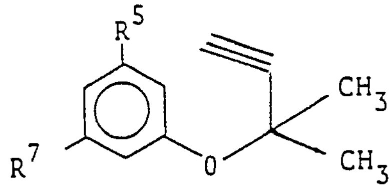

(IV)

dans laquelle $R^5$ et $R^7$ ont les significations indiquées ci-dessus.

2. Produit pesticide, caractérisé en ce qu'il comporte des dérivés du chromène de formule I

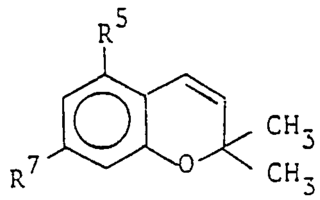

(I)

dans laquelle
$R^5$ représente un groupe méthyle ou méthoxy, et
$R^7$ représente un groupe alcoxy en C2-C4, cycloalcoxy en C3-C5 ou propargyloxy,
à titre de substances actives avec des véhicules solides et/ou des diluants liquides usuels et, le cas échéant, des substances tensioactives.

3. Produit pesticide selon la revendication 2, caractérisé en ce qu'il comporte des dérivés du chromène de formule II

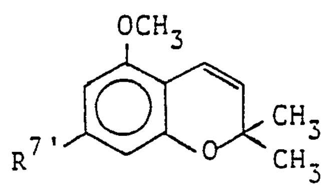

(II)

dans laquelle $R^{7'}$ représente un groupe n-propoxy, isopropoxy, sec-butoxy ou isobutoxy, à titre de substances actives avec des véhicules solides et/ou des diluants liquides usuels et, le cas échéant, des substances tensioactives.

4. Produit pesticide selon la revendication 2 caractérisé en ce qu'il comporte des dérivés du chromène de formule III

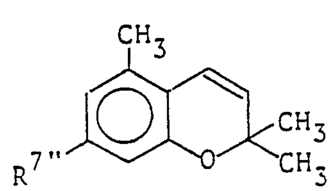

(III)

dans laquelle $R^{7''}$ représente un groupe éthoxy, isopropoxy, sec-butoxy ou n-cyclopentyloxy, à titre de substances actives avec des véhicules solides et/ou des diluants liquides usuels et, le cas échéant, des substances tensioactives.